(19) Europäisches Patentamt / European Patent Office / Office européen des brevets

(11) **EP 4 216 715 B1**

(12) **EUROPEAN PATENT SPECIFICATION**

(45) Date of publication and mention of the grant of the patent:
**04.09.2024 Bulletin 2024/36**

(21) Application number: **21773572.9**

(22) Date of filing: **08.09.2021**

(51) International Patent Classification (IPC):
***A01N 25/04*** (2006.01) ***A61K 8/31*** (2006.01)
***A61K 8/34*** (2006.01) ***A61K 8/81*** (2006.01)
***A61P 31/04*** (2006.01) ***A61Q 17/00*** (2006.01)
***A61Q 19/00*** (2006.01) ***A61K 47/00*** (2006.01)

(52) Cooperative Patent Classification (CPC):
(C-Sets available)
**A61K 8/31; A01N 25/04; A61K 8/34; A61K 8/345; A61K 8/8152; A61P 31/04; A61Q 17/005; A61Q 19/00;** A61K 2800/5922 (Cont.)

(86) International application number:
**PCT/EP2021/074689**

(87) International publication number:
**WO 2022/063576 (31.03.2022 Gazette 2022/13)**

(54) **MOISTURIZING ANTIBACTERIAL COMPOSITION**

FEUCHTIGKEITSSPENDENDE ANTIBAKTERIELLE ZUSAMMENSETZUNG

COMPOSITION ANTIBACTÉRIENNE HYDRATANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.09.2020 US 202063083570 P**

(43) Date of publication of application:
**02.08.2023 Bulletin 2023/31**

(73) Proprietors:
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO SE SI SK SM**
- **Unilever Global IP Limited**
  **Wirral, Merseyside CH62 4ZD (GB)**
  Designated Contracting States:
  **CY DE GB IE IT MT RS TR**

(72) Inventors:
- **CHANDAR, Prem**
  **Trumbull, CT Connecticut 06611 (US)**
- **DASGUPTA, Bivash, Ranjan**
  **Trumbull, CT Connecticut 06611 (US)**
- **FREY, Gabriella, Satchi, Olivia**
  **Trumbull, CT Connecticut 06611 (US)**
- **MOADDEL, Teanoosh**
  **Trumbull, CT Connecticut 06611 (US)**
- **SHILOACH, Anat**
  **Trumbull, CT Connecticut 06611 (US)**

(74) Representative: **Fijnvandraat, Arnoldus**
**Unilever Patent Group**
**Bronland 14**
**6708 WH Wageningen (NL)**

(56) References cited:
**EP-A1- 1 152 744**
**EP-A1- 3 595 621**
**EP-B1- 1 152 744**
**EP-B1- 1 784 074**
**US-A1- 2006 204 466**
**US-B2- 10 470 986**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(52) Cooperative Patent Classification (CPC): (Cont.)

C-Sets
**A01N 25/04, A01N 31/02**

## Description

### Field of the invention

Disclosed herein is an antibacterial composition, specifically, a moisturizing antibacterial composition. The moisturizing antibacterial composition includes water, alcohol, a thickening agent, and a moisturizing oil.

### Background of the invention

Consumers are increasingly concerned with microbial and viral contamination. As such, hygiene continues to be one of the most important attributes desired among consumers. Consumers desire products that will provide the required hygienic properties. Consumers throughout the world use different types of antibacterial compositions for disinfecting various surfaces including hard surfaces such as countertops, floors, and furniture; soft and porous surfaces such as clothes, carpets, and upholstery; and personal surfaces such as skin and hair. Many microorganisms like bacteria and viruses are found on such surfaces. It is desirable to keep these surfaces free of germs to minimize the risk of becoming ill.

Many antibacterial products are commercially available. Some are supplied as gels containing at least 60% by weight or more of ethanol. While such products are known to yield good antibacterial benefits after application, certain consumers shun using high ethanol-based products since such products may dry the skin, weaken the epidermis barrier, and expedite the aging process. In the current environment, consumers may be using antibacterial products on their bodies throughout the day thus increasing the likelihood of developing dry skin.

As such, there is continually a need to develop an antibacterial composition that provides excellent antimicrobial and antiviral benefits while at the same time being safe and mild enough to use an unlimited amount of times throughout the day. Given consumer demands, it is of increasing interest to develop an antibacterial composition that also delivers soothing and moisturizing benefits when topically applied.

### Summary of the invention

Disclosed in various aspects are moisturizing antibacterial compositions.

An antibacterial composition comprises: water; alcohol; a thickening agent; and a moisturizing oil, wherein at least 50% of droplets of moisturizing oil present in the antibacterial composition have a particle size of 50 micrometers to 2.5 millimeters.

A method of making an antibacterial composition comprises: dispersing a thickening agent in water forming a first phase; combining a moisturizing oil and a humectant forming a second phase; combining the first phase with an alcohol forming a third phase; adding the second phase to the third phase; and adding a neutralizer to the third phase, thereby forming the antibacterial composition.

These and other features and characteristics are more particularly described below.

### Detailed description of the invention

Disclosed herein is an antibacterial composition. The antibacterial composition is a moisturizing, opaque to transparent/translucent composition containing visible particulates with non-tacky sensory properties. The antibacterial composition can be any form, including, but not limited to, a liquid, gel, cream or lotion. The antibacterial composition provides moisturization to the surface onto which it is applied. For example, when the composition is applied to human skin, the antibacterial composition does not leave the user with dry skin, but rather with skin that has been both moisturized and sanitized. The antibacterial composition includes water, alcohol, a thickening agent, and a moisturizing oil. It was unexpectedly found that the specific moisturizing oil present in the antibacterial composition disclosed herein leaves the user with non-sticky, moisturized skin after application of the composition without requiring the use of a non-tacking agent.

The antibacterial composition can be substantially free of a non-tacking agent. Substantially free of a non-tacking agent means that the composition contains less than 2% by weight of a non-tacking agent, preferably, less than 1 % by weight of a non-tacking agent, more preferably less than 0.5% by weight of a non-tacking agent, even more preferably, less than 0.1 % by weight of a non-tacking agent, still more preterably, 0% by weight of a non-tacking agent. Percent by weight (% by weight) referred to herein throughout refers to the % by weight in the overall composition.

The antibacterial composition advantageously provides both moisturizing and sanitizing benefits to the surface onto which it is applied. Sanitizing or antibacterial composition as used herein refers to a composition that is capable of killing bacteria or inhibiting the growth of bacteria as often associated with compositions having at least 60% by weight alcohol. The antibacterial compositions can be substantially emulsifier free, opaque to transparent/translucent with visible particulates. Substantially emulsifier free means that the composition contains less than 3% by weight emulsifier, preferably, less than 2% by weight emulsifier, more preferably less than 0.5% by weight emulsifier. In some embodiments, the antibacterial composition contains no emulsifier or stated another way, 0% by weight emulsifier. Reducing the level of or eliminating the use of emulsifier can assist creating a more environmentally friendly composition without the use of an additional ingredient.

The antibacterial composition comprises water in an amount of 5 to 30% by weight, preferably 6 to 25% by weight, more preferably 7 to 20% by weight.

Alcohol is used as the antibacterial agent in the composition. The antibacterial composition comprises alcohol in an amount of 60 to 80% by weight, preferably 62 to 75% by weight, more preferably 65 to 75% by weight, including all values and ranges subsumed therein. The alcohol can comprise a variety of alcohols including, but not limited to, ethanol, propanol (e.g., n-propanol), isopropyl alcohol, or a combination thereof. Optionally, the alcohol can comprise chloroxylenol. A preferred alcohol can be denatured SD-40 ethanol.

The antibacterial composition can optionally comprise one or more additional antibacterial agents. The optional, additional antibacterial agent can be selected from terpenes, essential oils, or cationic oils having a solubility in water of less than 2000 parts per million (ppm) at 25°C. Examples of aromatic essential oils that can be used in the antibacterial compositions disclosed herein include amyl salicylate, carvacrol, cymene, e.g., p-cymene, dihydroeugenol, eugenol, hexyl eugenol, hexyl salicylate, isoeugenol, methyl eugenol, methyl isoeugenol, methyl salicylate, tert butyl cresol, thymol, and vanillin. Examples of non-aromatic essential oils of terpenoid compounds include cedrane, cineole, citral (including geranial and neral), citronellal, nitronelol, eucalyptol (i.e., 1,8 cineole) paradihydrolinalool, dihydromyrcenol (DH myrcenol), farnesol, geraniol, hexyl cinnamaldehyde, hydroxycitronallol, hydroxycitronellal, isocitral, limonene, preferably d-limonene, linallol, longifolene, menthol, nerol, nerolidiol, pinene, e.g., $\alpha$-pinene, phellendrene, terpinine, e.g., $\alpha$-terpinene and $\gamma$-terpinene, terpineol, e.g., $\gamma$-terpineol and terpin-4-ol, and tetrahydromyrcenol (THM).

Preferred cationic oils include quaternary ammonium cationic vegetable oil and charged aminopolydimethylsilane having the formula $(CH_3)_3$-Si[Si$(CH_3)_2$-O]-[Si$(CH_3)$-($(CH_2)_3$-NH-$(CH_2)_2$-$N_2$)-O$]_2$-Si-$(CH_3)_3$. It is preferred that the solubility of these additional antibacterial agents be less than 2000 ppm at 25°C. For example, the additional antibacterial agent can be terpineol, thymol, eugenol, borneol, limonene, or a combination thereof. A preferred additional antibacterial agent can be terpineol, thymol, or eugenol, or a combination thereof.

Other types of optional additional antibacterial agents include silver compounds. The silver compound can comprise a silver ion, for example, the silver ion can be selected from silver nitrate, silver acetate, silver oxide, silver sulfate, or a combination thereof. Preferably, the silver compound is silver nitrate.

Stated more specifically, the silver compounds optionally employed in the compositions are one or more water-soluble silver (I) compounds having a silver ion solubility of at least $1.0\times10^{-4}$ mol/L (in water at 25°C). Silver ion solubility, as referred to herein, is a value derived from a solubility product (Ksp) in water at 25°C, a well-known parameter that is reported in numerous sources. More particularly, silver ion solubility [Ag+], a value given in mol/L may be calculated using the formula:

$$[Ag+]=(Ksp\cdot x)^{(1/(x+1))},$$

wherein Ksp is the solubility product of the compound of interest in water at 25°C, and x represents the number of moles of silver ion per mole of compound. It has been found that silver (I) compounds having a silver ion solubility of at least $1\times10^{-4}$ mol/L are desirable for use herein.

Among the silver compounds desirable for use herein are silver oxide, silver nitrate, silver acetate, silver sulfate, silver benzoate, silver salicylate, silver carbonate, silver citrate and silver phosphate, or a combination thereof, preferably wherein the silver compound is silver nitrate, silver acetate, silver oxide, silver sulfate, or a combination thereof.

When present, the additional antibacterial agent can be included in an amount of 0.05 to 2% by weight, for example, 0.1 to 2% by weight of the overall antibacterial composition including all values and ranges subsumed therein.

A moisturizing oil can be used in the antibacterial composition to impart moisture to the surface to which it is applied. For example, when applied to human skin, the moisturizing oil can leave the skin feeling moisturized after application of the antibacterial composition and can assist in preventing excess drying of the skin typically associated with antibacterial compositions due to the antibacterial agent used in the composition.

The antibacterial composition comprises a moisturizing oil in an amount of 1 to 15% by weight, preferably 1 to 10% by weight, more preferably, 2 to 7% by weight including all values and ranges subsumed therein. The moisturizing oil can comprise an insoluble moisturizing oil. The moisturizing oil can have a minimum viscosity of 10 kiloPoise (kP) (10 Pascal*seconds (Pa*s)) at 32°C. Preferably the moisturizing oil can have a viscosity of 10 to 100 kP (10 to 100 Pa*s) at 32°C, more preferably, the upper limit of viscosity is 25 kP to 200 kP (25 to 200 Pa*s) at 32°C. The moisturizing oil can have a melting point of 35 to 90°C (determined according to ASTMD127-08).

In the antibacterial composition, droplets of the moisturizing oil present in the antibacterial composition can have a particle size of 5 micrometers ($\mu$m) to 10 millimeters (mm). A majority (i.e., greater than 50%) of the droplets of the moisturizing oil present in the antibacterial composition has a particle size of 50 μm to 2.5 mm. In an embodiment, a majority of (i.e., at least 50%) of the droplets in the moisturizing oil present in the antibacterial composition are visible to naked eye. For example, the particle size can be 100 μm to 1 mm. In the case when moisturizing oil is in the millimeter size range and visible to the naked eye the moisturizing oil can optionally contain dyes or pigments allowing the user to see the moisturizing oil clearly within the antibacterial composition.

Particle size as referred to herein can be measured using either visual or microscopic observation using an optical microscope fitted with cross-polarizers. Assessment of particle size can be accomplished by placing a sample between two glass plates and observing the sample through the microscope to assess particle size. Alternatively, a Mastersizer 3000 from Malvern Panalytical can be used. The Mastersizer uses laser diffraction to measure the particle sizes of a sample dispersed in water and then uses this information to generate a particle size distribution graph. Particle size is reported as D[4,3] which is the volume mean diameter.

To begin an analysis, the sample is dropped into the Mastersizer well with a pipet, the propeller at the bottom of the well is set to 2800 revolutions per minute (RPM), and the sample is allowed to thoroughly disperse (until a preview screen shows consistent peaks). After thoroughly dispersed, ten readings are taken of the particle distributions, and an average result is automatically calculated by the Mastersizer. Generally, this process is performed at least twice to ensure the sample was thoroughly dispersed.

The moisturizing oil can comprise petroleum jelly, natural based non-petroleum jelly, natural fats and oil, or a combination thereof. Natural based non-petroleum jelly for example could be plant or bio-derived.

Other possible, optional moisturizing oils present at total levels of less than about 2, 1.0, or 0.5% by wt. in the antimicrobial composition include but are not limited to the following:

(a) silicone oils and modifications thereof such as linear and cyclic polydimethylsiloxanes; amino, alkyl, alkylaryl, and aryl silicone oils;
(b) silicone elastomers such as dimethicone cross-polymers and hydrophobically modified dimethicone cross polymers
(c) fats and oils including natural fats and oils such as jojoba, soybean, sunflower, rice bran, avocado, almond, olive, sesame, persic, castor, coconut, mink oils; cacao fat; beef tallow, lard; hardened oils obtained by hydrogenating the aforementioned oils; and synthetic mono, di and triglycerides such as myristic acid glyceride and 2-ethylhexanoic acid glyceride;
(d) waxes such as carnauba, spermaceti, beeswax, lanolin, candelilla, microcrystalline and derivatives thereof;
(e) hydrophobic and hydrophillic plant extracts;
(f) hydrocarbons such as polybutene, liquid paraffins, ceresin, squalene, squalane, pristane and mineral oil;
(g) higher fatty acids such as lauric, myristic, palmitic, stearic, behenic, oleic, linoleic, linolenic, lanolic, isostearic, arachidonic and poly unsaturated fatty acids (PUFA);
(h) higher alcohols such as lauryl, cetyl, cetearyl, stearyl, oleyl, behenyl, cholesterol and 2-hexydecanol alcohol;
(i) esters such as cetyl octanoate, cetyl lactate, isopropyl myristate, myristyl myristate, isopropyl palmitate, isopropyl adipate, butyl stearate, decyl oleate, cholesterol isostearate, glycerol monostearate, glycerol distearate, glycerol tristearate, alkyl lactate, alkyl citrate and alkyl tartrate;
(j) essential oils and extracts thereof such as mentha, jasmine, camphor, white cedar, bitter orange peel, ryu, turpentine, cinnamon, bergamot, citrus unshiu, calamus, pine, lavender, bay, clove, hiba, eucalyptus, lemon, starflower, thyme, peppermint, rose, sage, sesame, ginger, basil, juniper, lemon grass, rosemary, rosewood, avocado, grape, grapeseed, myrrh, cucumber, watercress, calendula, elder flower, geranium, linden blossom, amaranth, seaweed, ginko, ginseng, carrot, guarana, tea tree, jojoba, comfrey, oatmeal, cocoa, neroli, vanilla, green tea, penny royal, aloe vera, menthol, cineole, eugenol, citral, citronelle, borneol, linalool, geraniol, evening primrose, camphor, thymol, spirantol, penene, limonene and terpenoid oils;
(k) combinations of any of the foregoing components, and the like.

In some embodiments, the composition is substantially free of silicone. Substantially free of silicone means that the composition contains less than 2% by weight of silicone, preferably, less than 1% by weight silicone, more preferably less than 0.5% by weight silicone, even more preferably, less than 0.1 % by weight silicone, still more preferably, 0% by weight silicone.

The antibacterial composition can additionally contain other ingredients in addition to those previously described herein including, but not limited to, skin benefit agents, fragrances, preservatives, surfactants, fixatives, opacifiers, chelators, thickening agents, humectants, thickening agents, or a combination thereof.

For example, humectants can optionally be used in the antibacterial composition to provide additional moisturization properties to the composition. Such humectants desirable for use in the antibacterial composition can include water soluble polyols such as propylene glycol, dipropylene glycol, polypropylene glycol (e.g., PPG-9), polyethylene glycol, hydroxypropyl sorbitol, hexylene glycol, 1 ,3-butylene glycol, 1 ,2-octane diol, 1,2-hexane diol, isoprene glycol, 1,2,6-hexanetriol, ethoxylated glycerol, propoxylated glycerol and combinations thereof. Most preferred are glycerin, butylene glycol, propylene glycol, polyethylene glycols, sorbitol, polyglycerol, isoprene glycol, or a combination thereof. The humectant can be present in an amount of 1 to 10% by weight, preferably 2 to 8% by weight, more preferably 3 to 7% by weight of the antibacterial composition.

To adjust antibacterial composition viscosity, it is within the scope of the compositions to optionally include thickening agents. Thickening agents can be used in the antibacterial composition to assist in forming the composition (e.g., giving it structure). Thickening agents can be used in an amount of 0.01% by weight to 2.5% by weight, for example, 0.1% by weight to 2.0% by weight, for example, 0.15% by weight to 1.5% by weight, for example, 0.2% by weight to 1.0% by weight including all values and ranges subsumed therein. The amount of thickening agent to be used can be adjusted based on the desired end use viscosity. For example as disclosed in the technical data sheet from Lubrizol for formulating hydroalcoholic gels with Carbopol® polymers edition dated September 3, 2009, typical end use polymer concentrations can range from 0.1-0.5% by weight resulting in viscosities ranging from 1 to 25,000 mPa*s, depending on the choice of the particular Carbopol® polymer being used.

Thickening agents can comprise anionic thickening agents, nonionic thickening agents, cationic thickening agents, or a combination thereof. For example, the thickening agent can comprise a hydrophobically modified crosspolymer. Synthetic polymers are effective thickening agents. Possible thickening agents include crosslinked polyacrylates such as the carbomers like ASHLAND™ 980 carbomers (cross linked polymer of acrylic acid), acrylate copolymers, acrylates/ acrylate ($C_{10}$-$C_{30}$) alkyl acrylate crosspolymers such as the Carbopol® line like Ultrez20 and ETD2020 commercially available from Lubrizol with an INCI name of acrylates/C 10-30 alkyl acrylate crosspolymer, still further Carbopo® series of polymers can also include Ultrez10, Ultrez21 and Aqua SF-1. Other thickening agents desirable for use can include polyacrylamides such as Sepigel® 305 and taurate copolymers such as Simulgel® EG and Aristoflex® AVC, the copolymers being identified by respective INCI nomenclature as sodium acrylate/sodium acryloyldimethyl taurate and acryloyl dimethyltaurate/vinyl pyrrolidone copolymer. Still other thickening polymers can include a synthetic polymer such as an acrylate-based polymer made commercially available by Seppic and sold under the name Simulgel INS100. Calcium carbonate, fumed silica, and magnesium-aluminum-silicate may also be used.

Still further thickening agents classified as polysaccharides can be used. Examples include fibers, starches, natural/synthetic gums and cellulosics. Representative of the starches are chemically modified starches such as sodium hydroxypropyl starch phosphate and aluminum starch octenylsuccinate. Tapioca starch is often preferred, as is maltodextrin. Gums include xanthan, tara, sclerotium, pectin, karaya, arabic, agar, guar (including Acacia senegal guar), carrageenan, alginate and combinations thereof. Cellulosics include hydroxypropyl cellulose, hydroxypropyl methylcellulose (e.g., BENECEL™ E10M by Ashland), ethylcellulose, sodium carboxy methylcellulose (cellulose gum/carboxymethyl cellulose) and cellulose (e.g. cellulose microfibrils, cellulose nanocrystals or microcrystalline cellulose). Sources of cellulose microfibrils include secondary cell wall materials (e.g. wood pulp, cotton), bacterial cellulose, and primary cell wall materials. Preferably the source of primary cell wall material is selected from parenchymal tissue from fruits, roots, bulbs, tubers, seeds, leaves and combination thereof; more preferably is selected from citrus fruit, tomato fruit, peach fruit, pumpkin fruit, kiwi fruit, apple fruit, mango fruit, sugar beet, beet root, turnip, parsnip, maize, oat, wheat, peas and combinations thereof; and even more preferably is selected from citrus fruit, tomato fruit and combinations thereof. A most preferred source of primary cell wall material is parenchymal tissue from citrus fruit. Citrus fibers, such as those made available by Herbacel® as AQ Plus can also be used as source for cellulose microfibrils. The cellulose sources can be surface modified by any of the known methods including those described in Colloidal Polymer Science, Kalia et al., "Nanofibrillated cellulose: surface modification and potential applications" (2014), Vol 292, Pages 5-31.

Other thickening agents often desired can also include maltodextrin, xanthan gum, and carboxymethyl cellulose, dilinoleyl/dimethyl carbonate copolymer, stearyl alkonium hectorite, tara gum, polyquaternium 32 and/or 37, pentaerythrityl tetrastearate or a combination thereof.

The antibacterial composition can optionally contain surfactants, for example, cationic surfactants. When used, the cationic surfactant is only limited in that it should be able to be used for topical application onto human skin. The cationic surfactant can comprise branched or straight chain alkyl trimonium compounds, alkanol trimonium compounds or a combination thereof. The alkanol trimonium compounds include lauroyl ethyltrimonium methosulfate, palmitoyl ethyltrimonium methosulfate, stearoyl ethyltrimonium methosulfate, carnitine, palmitoyl carnitine, a combination thereof or the like. The trimonium compound used can be an alkyl trimonium compound comprising cetrimonium chloride, cetrimonium bromide, mytrimonium chloride, mytrimonium bromide, behentrimonium methosulfate, cocotrimonium methosulfate, behentrimonium chloride, behentrimonium bromide, steartrimonium chloride, steartrimonium bromide, laurtrimonium chloride, laurtrimonium bromide, a combination thereof or the like. For the avoidance of doubt, cationic surfactant used in the antibacterial composition disclosed herein can consist essentially of or consist of any combination of the aforementioned surfactants.

As to the cationic surfactant comprising a dimonium compound, such a compound includes dialkyl dimonium compounds like distearyl dimonium chloride, didecyl dimonium chloride, dicoco dimonium chloride, a combination thereof or the like. Other dimonium compounds suitable for use include benzethonium chloride and/or benzalkonium chloride.

The dimonium and trimonium compounds used herein are meant to include salts of the same, especially chlorides and bromides of the same.

Polyquaternium materials can also be used and can include materials such as polyquaternium-6, polyquaternium-10, polyquaternium-16, polyquaternium-45, polyquaternium-28, polyquaternium-53, polyquaternium-67, acrylamidepropyl-trimonium chloride/acrylate (or acrylamide) copolymer, a combination thereof or the like.

The amount of cationic surfactant (i.e., cationic trimonium, dimonium) and/or polyquaternium material when used in the composition is typically 0.007 to 5% by weight, and preferably, from 0.01 to 3% by weight, and most preferably, from 0.05 to 1% by weight, based on total weight of the composition, including all values and ranges subsumed therein.

When both trimonium and dimonium surfactant are used they are often used in a weight ratio of 1:99 to 99:1, and preferably, from 30:70 to 70:30, and most preferably, from 40:60 to 60:40.

The antibacterial composition can also optionally include sunscreens and photostabilizers. The sunscreens and photostabilizers for use include such materials as octylmethoxycinnamate (OMC), ethylhexyl salicylate, phenylbenzimidazole sulfonic acid (Ensulizole), ethylhexyl p-methoxycinnamate, available as Parsol MCX®, Avobenzene (butyl methoxydibenzoylmethane), available as Parsol 1789® and benzophenone-3, also known as oxybenzone. Still others can inlcude bis-ethyl hexyloxyphenol methoxyphenol triazine, 2-ethylhexyl-2-cyano3,3-diphenyl-2-propanoic acid, drometrizole trisiloxane, 3,3,5-trimethyl cyclohexyl 2-hydroxybenzoate, 2-ethylhexyl-2-hydroxybenzoate or combination thereof. Inorganic sunscreen actives may be employed such as microfine titanium dioxide (preferably with a particle diameter of less than 150 nanometers (nm), and most preferably, less than 100 nm) and zinc oxide may be used, polyethylene and various other polymers are also suitable sunscreens. Other sunscreens suitable for use include p-aminobenzoic acid (PABA), octyldimethyl-PABA, 2-ethoxyethyl p-methoxy cinnamate, benzophenone-1, benzophenone-2, benzophenone-6, benzophenone-8, benzophenone-9, benzophenone-12, homomethyl salicylate, menthyl anthranilate, benzophenone-4, triethanolamine salicylate, terephthalylidene dicamphor sulfonic acid, bisoctriazole, bisethylhexyloxyphenol methoxyphenyl triazine, bisdisulizole disodium, diometriazole trisiloxane, octyltriazone, iscotrizinol, polysilicone-15, isopentenyl-4-methoxycinnamate, or a combination thereof. Octocrylene can also be used. Amounts of the sunscreen or photostabilizing agents when present can be 0.001 to 20%, preferably, 0.005 to 15%, optimally, 0.01 to 10%, or even 0.1 to 0.2% by weight of the antibacterial composition including all values and ranges subsumed therein.

Further optional water-soluble skin benefit agents suitable to use in the antibacterial composition disclosed herein include acids, such as amino acids like arginine, valine or histidine. Vitamins can be used such as vitamin $B_2$, picolinamide, niacinamide (vitamin $B_3$), panthenol (vitamin $B_5$), vitamin $B_6$, vitamin C, a combination thereof or the like. Derivatives, and especially, water soluble derivatives of such vitamins can also be employed. For instance, vitamin C derivatives such as ascorbyl tetraisopalmitate, magnesium ascorbyl phosphate and ascorbyl glycoside may be used alone or in combination with each other. Other skin benefit agents that can be used include hyaluronic acid and salts thereof (like Na+ and K+ salts of the same) 4-ethyl resorcinol, extracts like sage, aloe vera, green tea, sugar cane, citrus, grapeseed, thyme, chamomile, yarrow, cucumber, liquorice, rosemary extract or a combination thereof. Water soluble sunscreens like ensulizole may also be used as may electrolytes such as NaCl and/or KCl. The total amount of optional water-soluble benefit agents (including mixtures) when present in the composition disclosed herein can be 0.0001 to 10%, preferably, 0.001 to 6.5%, and most preferably, 0.01 to 3.5% by weight, based on total weight of the antibacterial composition and including all values and ranges subsumed therein.

It is also within the scope of the antibacterial composition to optionally include oil soluble benefit agents. Illustrative examples of the types of oil soluble benefit agents that can optionally be used in the antibacterial composition disclosed herein include components like stearic acid, vitamins like vitamin A, D, E and K (and their oil soluble derivatives).

For example, in a preferred embodiment, the composition can comprise Vitamin A in an amount of 0.001 to 0.75% by weight, preferably 0.01 to 0.5% by weight of the antibacterial composition, including all values and ranges subsumed therein.

Other optional oil soluble benefit agents for use include resorcinols like 4-hexyl resorcinol, 4-butyl resorcinol, 4-phenylethyl resorcinol, 4-cyclopentyl resorcinol, 4-cyclohexyl resorcinol 4-isopropyl resorcinol or a combination thereof. Also, 5-substituted resorcinols like 4-cyclohexyl-5-methylbenzene-1,3-diol, 4-isopropyl-5-methylbenzene-1 ,3-diol, combination thereof or the like may be used. The 5-substituted resorcinols, and their synthesis are described in commonly assigned U.S. Published Patent Application No. 2016/0000669A1.

Even other oil soluble benefit agents that can be used include omega-3 fatty acids, omega-6 fatty acids, climbazole, magnolol, honokiol, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, stearic acid, palmitic acid, lauric acid, terpineol, thymol essential components, the dissolution auxiliary selected from limonene, pinene, camphene, cymene, citronellol, citronellal, geraniol, nerol, linalool, rhodinol, borneol, isoborneol, menthone, camphor, safrole, isosafrole, eugenol, isoeugenol, tea tree oil, eucalyptus oil, peppermint oil, neem oil, lemon grass oil, orange oil, bergamot oil, or a combination thereof.

Another optional oil soluble benefit agent that may be used is a retinoic acid precursor. The retinoic acid precursor can be retinol, retinal, retinyl propionate, retinyl palmitate, retinyl acetate or a combination thereof. Retinyl propionate, retinyl palmitate and combinations thereof are typically preferred. Still another retinoic acid precursor for use is hydroxyanasatil retinoate made commercially available under the name Retextra® as supplied by Molecular Design International. The same may be used in a combination with any of the oil soluble benefit agents described herein.

When an optional (i.e., 0.0 to 1.5% by weight) oil soluble benefit agent is used in the antibacterial composition, it typically is present in an amount of 0.001 to 1.3%, and for example, 0.05 to 1.2%, for example, 0.1 to 0.5% by weight of the total weight of the end use composition, including all values and ranges subsumed therein.

Film forming agents may be used in the antibacterial compositions. While optional, such agents can aid with the composition adhering to the surface to which it is applied. Film forming agents include those having hydrophilic properties and they include materials comprising polyvinylpyrrolidone (PVP), acrylates, acrylamides, and copolymers thereof. Deposition agents like organosiloxanes and polyquaternium-7 (Merquat™ S Polymer from Lubrizol) may also be used. When used, such agents make up from 0.001 to 1% by weight of the antibacterial composition including all values and ranges subsumed therein.

Other optional components that can be used in the composition are anti-mosquito agents like eucalyptus oil, lavender oil, N,N-diethyl-meta-toluamide (DEET), a combination thereof or the like. Even other ingredients which may be used include octopirox (piroctone), zinc pyrithione, chloroxylenol, triclosan, cetylpyridinium chloride as well as silver compounds including silver oxide, nitrate, sulfate, phosphate, carbonate, acetate, benzoate, a combination thereof or the like. If used, these other components typically make up from 0.001 to 1.6%, and preferably, from 0.01 to 1.2% by weight of the antibacterial composition including all values and ranges subsumed therein.

Preservatives can optionally be used in the antibacterial composition disclosed herein. When used, illustrative preservatives include sodium benzoate, iodopropynyl butyl carbamate, phenoxyethanol, hydroxyacetophenone, ethylhexylglycerine, methyl paraben, propyl paraben, imidazolidinyl urea, sodium dehydroacetate, dimethyl-dimethyl (DMDM) hydantoin and benzyl alcohol or a combination thereof. Other preservatives suitable for use include sodium dehydroacetate, chlorophenesin and decylene glycol.

Preservatives are preferably employed in amounts of 0.01% to 2.0% by weight of the total weight of antibacterial composition, including all values and ranges subsumed therein. Also preferred is a preservative system with hydroxyacetophenone alone or in a mixture with other preservatives.

Fragrances, fixatives, opacifiers (like titanium dioxide), chelators (like EDTA) may optionally be included in the antibacterial composition. Each of these substances may be present in an amount of about 0.03 to about 3%, preferably, about 0.1 to about 2.6% by weight of the total weight of the antibacterial composition, including all values and ranges subsumed therein.

Another optional additive suitable for use includes hemp oil with 2.5 to 25% by weight cannabigerol and/or cannabidiol at from 0.5 to 10 percent by weight. When used, such oil makes up 0.0001 to 1.5% by weight of the antibacterial composition, and preferably, 0.01 to 1% by weight of the antibacterial composition, if used, including all values and ranges subsumed therein.

The antibacterial composition can optionally contain an emulsifier. The emulsifier may be selected from the group consisting of those with a $C_{10}$-$C_{20}$ fatty alcohol or acid hydrophobe condensed with about 2 to about 100 moles of ethylene oxide or propylene oxide per mole of hydrophobe; $C_2$-$C_{10}$ alkyl phenols condensed with 2 to 20 moles of alkylene oxide; mono- and di-fatty acid esters of ethylene glycol; sorbitan, mono- and di-$C_8$-$C_{20}$ fatty acids; and polyoxyethylene sorbitan, or a combinations thereof. Alkyl polyglycosides and saccharide fatty amides (e.g. methyl gluconamides) can also be used as nonionic emulsifiers.

When used, preferred emulsifiers typically have an HLB (hydrophilic-lipophilic balance) of 7.5 to 28, and preferably, 8 to 25, and most preferably, 9 to 20, including all values and ranges subsumed therein. e.g., nonionic emulsifier can include polysorbate 20 (Tween 20), polyoxyethylene (20) sorbitan monooleate (Tween 80). Other emulsifiers that can be used include emulsifying wax, cetearyl glucoside and combinations with cetearyl alcohol also known as Montanov 68, or a combination thereof. When present, the emulsifier can be present in an amount of 0 to 1% by weight, for example, 1% by weight including all values and ranges subsumed therein. As previously noted herein, the antibacterial compositions can be substantially emulsifier free and opaque to transparent/translucent. Substantially emulsifier free means that the composition contains less than 3% by weight emulsifier, preferably, less than 2% by weight emulsifier, more preferably less than 0.5% by weight emulsifier, most preferably, 0% by weight emulsifier including all values and ranges subsumed therein. Optionally, the emulsifier can comprise a phospholipid such as hydrogenated phosphatidylcholine (i.e., lecithin) in the emulsifier amounts previously described. In an embodiment, the antibacterial composition can be substantially free of hydrogenated phosphatidylcholine wherein substantially free refers to an amount of less than 0.05% by weight lecithin being present in the composition. In another embodiment, the antibacterial composition contains no lecithin (i.e., 0% by weight lecithin).

The antibacterial composition can additionally contain a neutralizing agent such as sodium hydroxide, potassium hydroxide, triethanolamine, ammonium, arginine, tromethamine, aminomethyl propanol sold as AMP 95, AMP-Ultra PC1000, AMP-Ultra PC2000, AMP-Ultra PC3000 by Angus, tetrahydroxylpropyl ethylenediamine also known as Neutrol TE from BASF, diisopropanolamine, triisopropanolamine, or a combination thereof. The neutralizing agent can be present in an amount of 0 to 1% by weight, for example, 0.1 to 0.75% by weight, for example, 0.2 to 0.5% by weight including all values and ranges subsumed therein. As disclosed in technical data sheet from Lubrizol dated edition September 16, 2009, the level of neutralization of a particular Carbopo® series of polymer, shown as variation in pH, can affect the final viscosity of the end use composition. If an anionic thickening agent is used, the level of neutralizer should be added and adjusted (increased/decreased) according to the amount of anionic thickener used to give a consumer acceptable end use viscosity at a skin friendly pH of between 3.5-8.2, more preferably 5-7.5.

Particulates, opacifiers and abrasives may also be included in compositions disclosed herein. Each of these substances can be present in an amount of about 0.05 to about 5%, preferably about 0.1 to about 3% by weight of the composition including all values and ranges subsumed therein.

As to packaging, the antibacterial composition, can be packaged in a spray bottle, squeeze bottle, or provided as an impregnating wetting agent on cotton swab, wipe, towelette, cosmetic substrate sheet (like those described in US 6,294,182 B1) or the like. As the viscosity is increased with thickening agent, the antibacterial composition gels and may be provided to consumers in a squeeze bottle as a gel composition. The spray bottle may also be metal, and the antibacterial composition may be provided via conventional aerosol packaging technologies and including those which utilize air-in-bag discharging cannisters, mechanisms and actuators. It is also within the scope of the antibacterial composition to include foaming agents (e.g., zwitterionic and/or amphoteric surfactants) so that the antibacterial composition can be discharged as a foam.

The antibacterial composition should be supplied with instructions to apply (e.g., squeeze or spray) the composition on to a surface, like skin, for bacteria kill and viral activity reduction. The antibacterial composition can be packaged in biodegradable packaging and the packaging used can be refillable or reusable, biodegradable, and/or at least 50%, and preferably, at least 100% made from post-consumer recycled resin.

Skin, as used herein, is meant to include skin on the arms (including underarms), face, feet, neck, chest, hands, legs, buttocks and scalp (including hair). Sanitizing as used herein means a bacteria log kill of at least 2 and a viral log inactivation of at least 2 (both achieved), preferably a bacteria log kill of at least 3 and a viral log inactivation of at least in less than 3 minutes after topical application to a surface. Combination, as used herein means, total weight, e.g., of cetrimonium chloride plus benzalkonium chloride. Skin benefit agent means an ingredient suitable to improve a skin characteristic. Surface as used herein includes skin or the surface of an inanimate object such as a tabletop, computer monitor, doorknob, toilet seat, shopping cart handle or even a clothing garment. Surface is also meant to include the coat of an animal such as the fur on a dog and cat. As used herein, surface preferably means human skin, and especially, skin on the face and hands.

The antibacterial composition can be a home care composition like a laundry spray composition suitable to spray clothing and upholstery requiring sanitizing. The home care composition can also be an antibacterial kitchen or bathroom spray composition.

Preferably, the antibacterial composition is a topical composition to apply to skin for sanitizing by significantly reducing the amount of bacteria and viruses on the skin. The composition may optionally comprise skin benefit ingredients added thereto such as emollients, vitamins and/or derivatives thereof, resorcinols, retinoic acid precursors, colorants, moisturizers or humectants, fragrances, sunscreens, a combination thereof or the like as previously described herein. The skin benefit ingredients may be water or oil soluble.

The antibacterial composition, therefore, is a hydroalcoholic based composition with a pH of 3.5 to 8.2, and the composition is water/alcohol continuous. Viscosity, as used herein, is taken either with a Brookfield viscometer using Spindle 4 at 10 rpm or with a Discovery HR-2 Rheometer using sand blasted plates having a 1000 micron gap and a first shear rate $S_A$ of 0.4 $s^{-1}$ for a first viscosity $V_A$ and a second shear rate $S_B$ of 10 $s^{-1}$ for a second viscosity $V_B$, both at 25°C and 20 second intervals. Viscosity is reported in centipoise (cps) (1000 centipoise (cps) = 1 Pascal second). In still another embodiment, the composition is a non-therapeutic and non-medicinal composition which is a water/alcohol continuous liquid having a viscosity under 30,000 centipoises (cps), preferably having a viscosity under 25,000 cps, preferably having a viscosity of 2,000-25,000 cps, more preferably having a viscosity of 2,000-10,000 cps.

Typically, the viscosity of the antibacterial composition will be under 30,000 cps. Often the viscosity of the antibacterial composition will be 0 to 25,000 cps, and preferably, 1500 to 25,000 cps, more preferably, 2,000 to 20,000 cps, and still more preferably, 2,000 to 10,000 cps, including all ranges subsumed therein.

The antibacterial compositions can be made by any method of making an antibacterial composition. The antibacterial composition is made at room temperature and the components are mixed until uniformity is reached. In one embodiment, a method of making the antibacterial composition disclosed herein can include the following: dispersing a thickening agent in water to form a first phase and combining a moisturizing oil and a humectant to form a second phase. The first phase is then combined with an alcohol to form a third phase and the second phase then added to the third phase. The antibacterial composition is then formed by adding a neutralizer to the third phase. A fragrance can then be added and/or optionally an opacifier.

Optionally EDTA can be used in the antibacterial compositions. EDTA, if present, can be included in the second phase.

The present antibacterial compositions are generally made in an ordinary or low shear mixing process without the requirements for high shear mixing or high pressure homogenization. The antibacterial compositions disclosed herein are able to be readily manufactured. The compositions are generally formed at atmospheric pressure and at 25°C.

## Examples

The following examples are merely illustrative of the antibacterial compositions disclosed herein and are not intended to limit the scope hereof.

Table 1 shows examples of antibacterial compositions as disclosed herein. The samples are made at room temperature, atmospheric pressure, and standard shear by dispersing a thickening agent in water to form phase A. Alcohol forms phase B. Then any skin benefit agents, humectants, and insoluble moisturizing oils are combined to form phase C. First, phases A and B are combined and mixed with an overhead pneumatic mixer until a clear mixture is formed at which point Phase C is added. The neutralizer is then added as Phase D and a fragrance added in Phase E thereby forming the antibacterial composition.

PJ1 refers to a natural non-petroleum jelly. The natural non-petroleum jelly can be derived from one or more of the following feedstocks: soy, rapeseed, coconut, palm, lard, tallow, cooking oil, having different chain length hydrocarbons. The natural non-petroleum jelly is not limited to the feedstocks listed herein but can include any natural or bio-derived feedstock. PJ2, in Sample 2, is prepared using a mixture of candelilla wax/soybean oil. PJ3, in Sample 3, is a vegetable derived based jelly from Sonneborn sold under the SonneNatural line. PJ4 is a standard petroleum jelly and is used in Sample 4. Sample 5 is prepared with PJ1 and no Tween 20. Sample 6 is prepared with PJ1 and with no Tween 20. Sample 7 is prepared with PJ1 and with no Tween 20.

Table 1

| **Sample #** | **1** | **2** | **3** | **4** | **5** | **6** | **7** |
|---|---|---|---|---|---|---|---|
| **Ingredient** | | | | | | | |
| **Water** | 8.9 | 13.65 | 11.8 | 12.85 | 16.9 | 14.9 | 16.55 |
| **Acrylates/C10-30 Alkyl Acrylate crosspolymer** | 0.4 | 0.45 | 0.4 | 0.45 | 0.3 | 0.35 | 0.5 |
| **Alcohol (Ethanol) / SDA Alcohol 40B 190 Proof (Pharmco-Aaper): Ethanol 95% denat 6ppm bitrex 0.12% tba** | 72 | 67 | 72 | 70 | 70 | 72 | 72 |
| **Isopropyl alcohol** | --- | 5 | --- | --- | --- | --- | --- |
| **Glycerin** | 7 | 7 | 5 | 3 | 5 | 5 | 5 |
| **Butylene Glycol** | --- | --- | 3 | 2 | 2 | 2 | --- |
| **Emulsifier (Tween 20)** | 1 | 1 | 1 | 1 | --- | --- | --- |
| **Aminomethyl propanol** | 0.25 | 0.4 | 0.3 | 0.2 | 0.3 | 0.2 | 0.45 |
| **Fragrance** | 0.5 | 0.5 | 0.5 | 0.5 | 0.5 | 0.5 | 0.5 |
| **PJ1** | 10 | --- | --- | --- | --- | 5 | 5 |
| **PJ2** | --- | 5 | --- | --- | --- | --- | --- |
| **PJ3** | --- | --- | 6 | --- | --- | --- | --- |
| **PJ4** | --- | --- | --- | 10 | 5 | --- | --- |

Without wishing to be bound by theory, it is believed that the antibacterial compositions disclosed herein are able to accommodate high levels of petroleum jelly and yet maintain stability for a period of time such as after 3 months at 40°C with no appreciable drop in viscosity over time or visible phase separation in the composition.

In the absence of explicitly stating otherwise, all ranges described herein are meant to include all ranges subsumed therein. As used herein, except where explicitly described, substantially free of means less than 10% by weight. Antimicrobial benefits mean at least a log kill of 2, preferably at least a log kill of 3, in under 3 minutes whereby antimicrobial assessment is measured via ASTM International standard method E2783-11 (Reapproved 2016) which sets forth the procedure for measuring antimicrobial activity for water miscible compounds using a time kill procedure. Viral (or virus) inactivation is determined by assessing the impact of microbiocides against viruses as set forth in ASTM International standard method 1052-20. The term comprising is meant to encompass the terms consisting essentially of and consisting of. For the avoidance of doubt, and for illustration, a composition comprising water, cationic surfactant and preservative is meant to include a composition consisting essentially of the same and a composition consisting of the same.

Except where otherwise explicitly indicated, all numbers in this description indicating amounts of material or conditions of reaction, physical properties of materials and/or use are to be understood as modified by the word "about." All amounts are by weight of the final composition, unless otherwise specified.

It should be noted that in specifying any range of concentration or amount, any particular upper concentration can be associated with any particular lower concentration or amount as well as any subranges consumed therein. In that regard, it is noted that all ranges disclosed herein are inclusive of the endpoints, and the endpoints are independently combinable with each other (e.g., ranges of "up to 25% by weight, or, more specifically, 5% by weight to 20% by weight, in inclusive of the endpoints and all intermediate values of the ranges of 5% by weight to 25% by weight, etc.). "Combination is inclusive of blends, mixtures, alloys, reaction products, and the like. Furthermore, the terms "first", "second", and the like herein do not denote any order, quantity, or importance, but rather are used to distinguish one element from another. The terms "a" and "an" and "the" herein do not denote a limitation of quantity and are to be construed to cover both the singular and the plural, unless otherwise indicated herein or clearly contradicted by context. The suffix "(s)" as used herein is intended to include both the singular and the plural of the term it modifies, thereby including one or more of the term (e.g., the film(s) includes one or more films). Reference throughout the specification to "one embodiment", "one aspect", "another embodiment", "another aspect", "an embodiment", "an aspect" and so forth means that a particular element (e.g., feature, structure, and/or characteristic) described in connection with the embodiment or aspect is included in at least one embodiment or aspect described herein and may or may not be present in other embodiments or aspects. In addition, it is to be understood that the described elements may be combined in any suitable manner in the various embodiments or aspects.

All cited patents, patent applications, and other references are incorporated herein by reference in their entirety. However, if a term in the present application contradicts or conflicts with a term in the incorporated reference, the term from the present application takes precedence over the conflicting term from the incorporated reference. While particular aspects have been described, alternatives, modifications, variations, improvements, and substantial equivalents that are or may be presently unforeseen may arise to applicants or others skilled in the art. Accordingly, the appended claims as filed and as they may be amended are intended to embrace all such alternatives, modifications, variations, improvements, and substantial equivalents.

For the avoidance of doubt the word "comprising" is intended to mean "including" but not necessarily "consisting of" or "composed of." In other words, the listed steps, options, or alternatives need not be exhaustive.

The disclosure of the invention as found herein is to be considered to cover all aspects as found in the claims as being multiply dependent upon each other irrespective of the fact that claims may be found without multiple dependency or redundancy. Unless otherwise specified, numerical ranges expressed in the format "from x to y" are understood to include x and y. In specifying any range of values or amounts, any particular upper value or amount can be associated with any particular lower value or amount. All percentages and ratios contained herein are calculated by weight unless otherwise indicated. The various features of the present invention referred to in individual sections above apply, as appropriate, to other sections *mutatis mutandis.* Consequently, features specified in one section may be combined with features specified in other sections as appropriate. Any section headings are added for convenience only and are not intended to limit the disclosure in any way.

## Claims

**1.** An antibacterial composition, comprising:

water;
alcohol, wherein the alcohol is present in an amount of 60 to 80% by weight of the overall antibacterial composition;
a thickening agent; and
a moisturizing oil, wherein at least 50% of droplets of the moisturizing oil present in the antibacterial composition have a D[4,3] particle size of 50 micrometers to 2.5 millimeters.

**2.** The antibacterial composition of Claim 1, wherein the moisturizing oil comprises an insoluble moisturizing oil.

**3.** The antibacterial composition of Claim 1 or Claim 2, wherein the moisturizing oil has a minimum viscosity of 10 kPa·s at 32°C.

**4.** The antibacterial composition of any of the preceding claims, wherein the moisturizing oil comprises petroleum jelly, natural non-petroleum jelly, natural fats and oils, or combinations thereof.

**5.** The antibacterial composition of any of the preceding claims, wherein the water is present in an amount of 5 to 30% by weight of the overall antibacterial composition, preferably 6 to 25% by weight, more preferably 7 to 20% by weight.

**6.** The antibacterial composition of any of the preceding claims, wherein the alcohol is preferably present in an amount of 62 to 75% by weight, more preferably 65 to 75% by weight.

**7.** The antibacterial composition of any of the preceding claims, wherein the moisturizing oil is present in an amount of 1 to 15% by weight of the overall antibacterial composition, preferably, 1 to 10% by weight, more preferably, 2 to 7% by weight, still more preferably, 3 to 5% by weight.

**8.** The antibacterial composition of any of the preceding claims, further comprising a humectant, wherein the humectant is present in an amount of 1 to 10% by weight, preferably 2 to 8% by weight, more preferably 3 to 7% by weight.

**9.** The antibacterial composition of Claim 8, wherein the humectant comprises glycerin, butylene glycol, propylene glycol, polyethylene glycols, sorbitol, polyglycerol, isoprene glycol, or a combination thereof.

**10.** The antibacterial composition of any of the preceding claims, wherein the thickening agent comprises an anionic thickening agent, a nonionic thickening agent, a cationic thickening agent, or a combination thereof.

**11.** The antibacterial composition of Claim 10, wherein the thickening agent comprises a hydrophobically modified acrylate crosspolymer.

**12.** A method of making an antibacterial composition, comprising:

dispersing a thickening agent in water to form a first phase;
combining a moisturizing oil and a humectant to form a second phase, wherein at least 50% of droplets of the moisturizing oil present in the antibacterial composition have a D[4,3] particle size of 50 micrometers to 2.5 millimeters;
combining the first phase with an alcohol to form a third phase;
adding the second phase to the third phase, forming a fourth phase; and
adding a neutralizer to the fourth phase, thereby forming the antibacterial composition.

**13.** The method of Claim 12, wherein the thickening agent comprises an anionic thickening agent, a nonionic thickening agent, a cationic thickening agent or a combination thereof.

**14.** The method of any of Claims 12 to 13, wherein the moisturizing oil comprises petroleum jelly, natural non-petroleum jelly, natural fats and oils, or combinations thereof.

## Patentansprüche

**1.** Antibakterielle Zusammensetzung, umfassend:

Wasser;
Alkohol, wobei der Alkohol in einer Menge von 60 bis 80 Gewichts-% der gesamten antibakteriellen Zusammensetzung vorliegt;
ein Verdickungsmittel; und
ein feuchtigkeitsspendendes Öl, wobei mindestens 50% der Tröpfchen des in der antibakteriellen Zusammensetzung vorliegenden feuchtigkeitsspendenden Öls eine D[4,3]-Partikelgröße von 50 Mikrometer bis 2,5 Millimeter aufweisen.

**2.** Antibakterielle Zusammensetzung nach Anspruch 1, wobei das feuchtigkeitsspendende Öl ein unlösliches feuchtigkeitsspendendes Öl ist.

**3.** Antibakterielle Zusammensetzung nach Anspruch 1 oder Anspruch 2, wobei das feuchtigkeitsspendende Öl bei 32°C eine Mindestviskosität von 10 kPa.s aufweist.

4. Antibakterielle Zusammensetzung nach einem der vorhergehenden Ansprüche, wobei das feuchtigkeitsspendende Öl Vaseline, natürliche Vaseline, natürliche Fette und Öle oder Kombinationen davon umfasst.

5. Antibakterielle Zusammensetzung nach einem der vorhergehenden Ansprüche, wobei das Wasser in einer Menge von 5 bis 30 Gewichts-% der gesamten antibakteriellen Zusammensetzung, vorzugsweise von 6 bis 25 Gewicht-%, bevorzugter von 7 bis 20 Gewichts-%, vorliegt.

6. Antibakterielle Zusammensetzung nach einem der vorhergehenden Ansprüche, wobei der Alkohol vorzugsweise in einer Menge von 62 bis 75 Gewichts-%, bevorzugter von 65 bis 75 Gewichts-%, vorliegt.

7. Antibakterielle Zusammensetzung nach einem der vorhergehenden Ansprüche, wobei das feuchtigkeitsspendende Öl in einer Menge von 1 bis 15 Gewichts-% der gesamten antibakteriellen Zusammensetzung, vorzugsweise von 1 bis 10 Gewichts-%, bevorzugter von 2 bis 7 Gewichts-%, noch bevorzugter von 3 bis 5 Gewichts-%, vorliegt.

8. Antibakterielle Zusammensetzung nach einem der vorhergehenden Ansprüche, die außerdem ein Feuchthaltemittel enthält, wobei das Feuchthaltemittel in einer Menge von 1 bis 10 Gewichts-%, vorzugsweise von 2 bis 8 Gewichts-%, bevorzugter von 3 bis 7 Gewichts-%, vorliegt.

9. Antibakterielle Zusammensetzung nach Anspruch 8, wobei das Feuchthaltemittel Glycerin, Butylenglykol, Propylenglykol, Polyethylenglykole, Sorbitol, Polyglycerin, Isoprenglykol oder eine Kombination davon umfasst.

10. Antibakterielle Zusammensetzung nach einem der vorhergehenden Ansprüche, wobei das Verdickungsmittel ein anionisches Verdickungsmittel, ein nichtionisches Verdickungsmittel, ein kationisches Verdickungsmittel oder eine Kombination davon umfasst.

11. Antibakterielle Zusammensetzung nach Anspruch 10, wobei das Verdickungsmittel ein hydrophob modifiziertes Acrylat-Crosspolymer umfasst.

12. Verfahren zur Herstellung einer antibakteriellen Zusammensetzung, umfassend:

Dispergieren eines Verdickungsmittels in Wasser, um eine erste Phase zu bilden;
Kombinieren eines feuchtigkeitsspendenden Öls und eines Feuchthaltemittels, um eine zweite Phase zu bilden, wobei mindestens 50% der Tröpfchen des in der antibakteriellen Zusammensetzung vorliegenden feuchtigkeitsspendenden Öls eine D[4,3]-Partikelgröße von 50 Mikrometer bis 2,5 Millimeter aufweisen;
Kombinieren der ersten Phase mit einem Alkohol, um eine dritte Phase zu bilden;
Hinzufügen der zweiten Phase zu der dritten Phase, um eine vierte Phase zu bilden, und
Hinzufügen eines Neutralisators zu der vierten Phase, wodurch die antibakterielle Zusammensetzung gebildet wird.

13. Verfahren nach Anspruch 12, wobei das Verdickungsmittel ein anionisches Verdickungsmittel, ein nichtionisches Verdickungsmittel, ein kationisches Verdickungsmittel oder eine Kombination davon umfasst.

14. Verfahren nach einem der Ansprüche 12 bis 13, wobei das feuchtigkeitsspendende Öl Vaseline, natürliche Vaseline, natürliche Fette und Öle oder Kombinationen davon umfasst.

## Revendications

1. Composition antibactérienne comprenant :

de l'eau ;
un alcool, lequel alcool est présent en une quantité de 60 à 80 % en poids de la composition antibactérienne totale ;
un agent épaississant ; et
une huile hydratante, dans laquelle au moins 50 % des gouttelettes de l'huile hydratante présente dans la composition antibactérienne ont une granulométrie D[4,3] de 50 micromètres à 2,5 millimètres.

2. Composition antibactérienne selon la revendication 1, dans laquelle l'huile hydratante comprend une huile hydratante insoluble.

3. Composition antibactérienne selon la revendication 1 ou la revendication 2, dans laquelle l'huile hydratante a une viscosité minimale de 10 kPa.s à 32°C.

4. Composition antibactérienne selon l'une quelconque des revendications précédentes, dans laquelle l'huile hydratante comprend de la gelée de pétrole, une gelée naturelle non de pétrole, des graisses et huiles naturelles, ou leurs combinaisons.

5. Composition antibactérienne selon l'une quelconque des revendications précédentes, dans laquelle l'eau est présente en une quantité de 5 à 30 % en poids de la composition antibactérienne totale, de préférence de 6 à 25 % en poids, mieux encore de 7 à 20 % en poids.

6. Composition antibactérienne selon l'une quelconque des revendications précédentes, dans laquelle l'alcool est de préférence présent en une quantité de 62 à 75 % en poids, mieux encore de 65 à 75 % en poids.

7. Composition antibactérienne selon l'une quelconque des revendications précédentes, dans laquelle l'huile hydratante est présente en une quantité de 1 à 15 % en poids de la composition antibactérienne totale, de préférence de 1 à 10 % en poids, mieux encore de 2 à 7 % en poids, plus particulièrement de 3 à 5 % en poids.

8. Composition antibactérienne selon l'une quelconque des revendications précédentes, comprenant en outre un humectant, dans laquelle l'humectant est présent en une quantité de 1 à 10 % en poids, de préférence de 2 à 8 % en poids, mieux encore de 3 à 7 % en poids.

9. Composition antibactérienne selon la revendication 8, dans laquelle l'humectant comprend du glycérol, du butylèneglycol, du propylèneglycol, des polyéthylèneglycols, du sorbitol, du polyglycérol, de l'isoprèneglycol, ou une de leurs combinaisons.

10. Composition antibactérienne selon l'une quelconque des revendications précédentes, dans laquelle l'agent épaississant comprend un agent épaississant anionique, un agent épaississant non-ionique, un agent épaississant cationique, ou une de leurs combinaisons.

11. Composition antibactérienne selon la revendication 10, dans laquelle l'agent épaississant comprend un polymère réticulé d'acrylate à modification hydrophobe.

12. Procédé de préparation d'une composition antibactérienne, comprenant :

la dispersion d'un agent épaississant dans de l'eau pour former une première phase ;
la combinaison d'une huile hydratante et d'un humectant pour former une deuxième phase, dans laquelle au moins 50 % des gouttelettes de l'huile hydratante présente dans la composition antibactérienne ont une granulométrie D[4,3] de 50 micromètres à 2,5 millimètres ;
la combinaison de la première phase avec un alcool pour former une troisième phase ;
l'addition de la deuxième phase à la troisième phase, ce qui forme une quatrième phase ; et
l'addition d'un neutralisant à la quatrième phase, ce qui forme ainsi la composition antibactérienne.

13. Procédé selon la revendication 12, dans lequel l'agent épaississant comprend un agent épaississant anionique, un agent épaississant non-ionique, un agent épaississant cationique ou une de leurs combinaisons.

14. Procédé selon l'une quelconque des revendications 12 et 13, dans lequel l'huile hydratante comprend de la gelée de pétrole, une gelée naturelle non de pétrole, des graisses et huiles naturelles, ou leurs combinaisons.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20160000669 A1
- US 6294182 B1

### Non-patent literature cited in the description

- **KALIA et al.** Nanofibrillated cellulose: surface modification and potential applications. *Colloidal Polymer Science,* 2014, vol. 292, 5-31